# EUROPEAN PATENT APPLICATION

(11) **EP 3 656 319 A1**
(43) Date of publication of application: **27.05.2020**
(21) Application number: 18835786.7
(22) Date of filing: 09.07.2018
(51) Int. Cl.: A61B 17/32

(54) **REUSABLE ULTRASONIC SURGICAL INSTRUMENT**

(30) Priority: 21.07.2017 CN 201710602001
(71) Applicant: Shanghai Yisi Medical Technology Co., Ltd., Shanghai 201318 (CN); Yisi Suzhou Medical Technology Co., Ltd., Suzhou City, Jiangsu 215163 (CN)
(72) Inventor: LI, Guoxin, Shanghai 201318 (CN); LI, Zhidong, Shanghai 201318 (CN); ZHENG, Yubin, Shanghai 201318 (CN); SUN, Changjiang, Shanghai 201318 (CN); ZHANG, Yu, Shanghai 201318 (CN); DING, Hao, Shanghai 201318 (CN); LIU, Ruixuan, Shanghai 201318 (CN); CHANG, Wangtao, Shanghai 201318 (CN)
(74) Representative: Strachan, Victoria Jane
(86) International application number: PCT/CN2018/094989
(87) International publication number: WO 2019/015502

(57) **Abstract**

The present application provides a reusable ultrasonic surgical instrument, comprising a cannula subassembly, a scalpel waveguide, a base and a clamping driving component, wherein the cannula subassembly comprises a pair of clamp forceps at a distal end, an external cannula and an internal cannula, and the external cannula and the internal cannula are both provided by being coaxial with the scalpel bar; two rotating shafts are provided at a proximal end of the clamp forceps, one of the two rotating shafts is rotatably connected with the external cannula, the other rotating shaft is rotatably connected with the internal cannula; the cannula subassembly can be dismounted from the base and the clamping driving component towards a distal end direction of the axis of the scalpel bar or be mounted on the base or the clamping driving component towards a proximal end direction of the axis of the scalpel bar; the external cannula is connected with the base by one or multiple first detachable structures; and the internal cannula is connected with the clamping driving component by one or multiple second detachable structures. The present application has the advantages of being simple in structure and low in cost.

## Description

### TECHNICAL FIELD

The present application relates to a surgical instrument, and particularly relates to a reusable ultrasonic surgical instrument.

### BACKGROUD

Along with popularization of minimally invasive surgery, ultrasonic scalpels have already become a kind of conventional surgical instrument. According to an ultrasonic scalpel, a scalpel head performs mechanical vibration at a certain ultrasonic frequency via an ultrasonic generator, so that water molecules in tissues evaporate, protein hydrogen bonds rupture and cells disintegrate, and then the tissues are cut open or coagulated, and also blood vessels are closed. Ultrasonic scalpels complete tissue cutting and coagulation and hemostasis at the same time, and have very small lateral heat injury.

An ultrasonic scalpel system is mainly formed by an ultrasonic generator, a transducer and a surgical instrument. The ultrasonic generator emits an oscillating electric signal, the transducer converts the oscillating electric signal into mechanical vibration, and the surgical instrument performs cutting and coagulation and hemostasis on a tissue by utilizing the mechanical vibration of the transducer. A surgical instrument is generally formed by a scalpel waveguide, a pair of clamp forceps forming a clamping structure with a scalpel head (a cutting portion at the head of the scalpel bar), a cannula encircling the outside of the scalpel bar, a grab handle and a grasping mechanism. The scalpel bar transmits mechanical vibration generated by the transducer to the scalpel head; the scalpel head is matched with the clamp forceps to clamp a tissue to realize the functions of cutting and coagulation and hemostasis; the cannula isolates the scalpel bar from the outside to play a role of protecting the scalpel bar on one hand, and forms a link mechanism with the clamp forceps to drive the closing and opening of the clamp forceps on the other hand; the grab handle and the grasping mechanism are held by the hand of a doctor to operate the clamp forceps to open and close, and a switch is provided to control the ultrasonic generator to start to output an oscillating electric signal or stopping outputting an oscillating electric signal.

For mainstream ultrasonic scalpel systems on the market currently, such as that disclosed in CN101035482 B (Reference 1) and CN106028979 A (Reference 2), a tube of a surgical instrument is formed by an external cannula and an internal cannula, and a scalpel waveguide is located in the internal cannula. Clearances between the internal cannula and the external cannula and between the internal cannula and the scalpel bar are small. After completing one surgery, some blood or tissue fluid may still enter the clearances between the internal cannula and the external cannula or between the internal cannula and the scalpel bar, and because the clearances are very small and narrow, blood or tissue fluid entering the clearances are hard to be thoroughly cleaned. Therefore, the surgical instrument cannot be used repeatedly even if the structure and performances are still good, and may be only used as a disposable instrument, therefore, the use cost is very high.

In order to realize repeated use of the surgical instrument to lower the use cost, CN202908793 U (Reference 3) and CN203988246 U (Reference 4) proposed corresponding technical schemes. In CN202908793 U (Reference 3), a cannula, a scalpel waveguide and clamp forceps are designed to be a removable subassembly structure, which can be used by only one time, while other components can be repeatedly used for many times. Although such design lowers the cost to a certain extent, however, because cost of these components including the cannula, the scalpel bar and the clamp forceps occupies a major portion of the cost of the surgical instrument, cost reduction is limited. A technical scheme adopted in CN203988246 U (Reference 4) is that a cannula and clamp forceps are designed to be removable subassemblies, can be used by only one time, while other components, including a scalpel waveguide, all can be repeatedly used for many times, which can effectively lower the use cost. However, components involved in the technical scheme are more, more processes such as welding and threaded connection are adopted, and thus implementation is more complicated.

### References

[1] CN101035482 B, name of the invention being *Ultrasonic surgical instrument;*
[2] CN106028979 A, name of the invention being *Clamping Arm Feature Structure Used for Ultrasonic Surgical Instrument;*
[3] CN202908793 U, name of the invention being *Ultrasonic Scalpel with Replaceable Scalpel Bar;*
[4] CN203988246 U, name of the invention being *Detachable Ultrasonic Scalpel Tube Rapid Docking Assembly.*

### SUMMARY

In order to solve the foregoing technical problem, the present application provides a reusable ultrasonic surgical instrument, which has the advantages of being simple in structure, easy in process realization, convenient in operation and low in cost in comparison with the prior art.

According to an aspect of the present application, a reusable ultrasonic surgical instrument is provided, including a cannula subassembly, a scalpel waveguide, a base and a clamping driving component, the cannula subassembly including a pair of clamp forceps at a distal end, an external cannula and an internal cannula, and the external cannula and the internal cannula being both provided by being coaxial with the scalpel bar; two rotating shafts being provided at a proximal end of the clamp forceps, one of the two rotating shafts being rotatably connected with the external cannula, the other rotating shaft being rotatably connected with the internal cannula; the cannula subassembly being dismounted from the base and the clamping driving component towards a distal end direction of the axis of the scalpel bar or being mounted on the base or the clamping driving component towards a proximal end direction of the axis of the scalpel bar; the external cannula being connected with the base by one or more first detachable structures; and the internal cannula being connected with the clamping driving component by one or more second detachable structures.

In an implementation mode, the first detachable structure is formed by an unthreaded hole in the base, a threaded hole in the external cannula and a bolt, the bolt being capable of penetrating through the unthreaded hole in the base to be screwed into or screwed out from the threaded hole in the external cannula.

In another implementation mode, the first detachable structure is formed in a threaded hole in the base, an unthreaded hole in the external cannula and a bolt, the bolt being screwed into or screwed out from the threaded hole in the base to penetrate into or withdraw from the unthreaded hole in the external cannula.

In another implementation mode, the first detachable structure is formed by a first clamping slot, a button, an elastic element and a clamping seat; the first clamping slot being formed by mutual connection of a first sliding slot and a first stop slot; the button having a button axis, and comprising a mounting section, a stop section and a dismounting section which are distributed along the axis direction of the button and are connected with one another; the elastic element being provided in the button axis direction of the button so that the button can move between a highest position and a lowest position along the button axis direction; the mounting section of the button being connected with the clamping seat and the elastic element, so that the button is in the highest position in a natural state, and can move to the lowest position by overcoming the elastic movement of the elastic element under the action of an external force; the stop section of the button being embedded into the first stop slot, and being restrained by the first stop slot in movement vertical to the direction of the button axis; and the dismounting section of the button being capable of freely sliding in the first clamping slot.

Further, the first clamping slot is provided on the base, and the button, the elastic element and the clamping seat are provided on the external cannula.

In an implementation mode, the first clamping slot is provided on the external cannula, and the button, the elastic element and the clamping seat are provided on the base.

Further, the clamping seat is a hole with a certain height in the base or the external cannula, and the mounting section of the button is mounted in the clamping seat by the elastic element and can move between a highest position and a lowest position along the hole.

Further, one end of the elastic element is pressed at the bottom of the mounting section of the button, and the other end is pressed on a cylindrical component sealing the bottom of the clamping seat.

In another implementation mode, the button is connected with the clamping seat by a structure of a buckle and a buttonhole, the height of the buttonhole being greater than the height of the buckle, so that the buckle can move in the buttonhole along the button axis direction, and then the button can move between a highest position and a lowest position.

Further, the buckle is provided on the clamping seat, and the buttonhole is formed in the button; or the buckle is provided on the button, and the buttonhole is formed in the clamping seat.

Further, the elastic element is a cylinder spring, a wave spring or other elastomers.

In another implementation mode, a part of the first sliding slot is parallel to the axis of the scalpel bar, and the other part is vertical to the axis of the scalpel bar; and the maximum width of the first stop slot is greater than the maximum width of the first sliding slot.

In any of the foregoing implementation modes, the second detachable structure is formed by a second clamping slot and a hasp structure. The second clamping slot is formed by mutual connection of a second sliding slot and a second stop slot; and the hasp structure is capable of freely sliding in the second sliding slot and sliding into the second stop slot from the second sliding slot, and is restrained by the second stop slot in movement along the axis direction of the scalpel bar.

Further, the second clamping slot is located on the internal cannula, and the hasp structure is located on the clamping driving component.

In another implementation mode, the second clamping slot is located on the clamping driving component, and the hasp structure is located on the internal cannula.

In an implementation mode, the second sliding slot is parallel to the axis of the scalpel bar.

Further, the second stop slot is vertical to the axis of the scalpel bar.

For the reusable ultrasonic surgical instrument according to the present application, the cannula subassembly is detachably connected with the scalpel bar, the base and the clamping driving component, so as to be convenient to dismount for cleaning after use, and then can be repeatedly used, thereby solving the problems that mainstream ultrasonic surgical instruments on the market are difficult to clean and cannot be repeatedly used, and remarkably lowering the use cost of the instrument. Moreover, in comparison with a reusable ultrasonic system of Reference 3, non-reusable components in the reusable ultrasonic surgical instrument of the present application are few, so as to further lower the use cost, and in comparison with Reference 4, the present application is promoted in the aspects of dismounting convenience, reliability of overall structure, and simplicity in process realization. In brief, the present application has the advantages of being simple in structure and low in cost in comparison with the prior art.

### BRIEF DESCRIPTION OF THE DRAWINGS

FIG. 1 is a schematic diagram of an ultrasonic scalpel system;
FIG. 2 is a schematic diagram of a reusable surgical instrument according to a first implementation mode of the present application;
FIG. 3 is a schematic diagram of a second detachable structure in FIG. 2;
FIG. 4 is a schematic diagram of a reusable ultrasonic surgical instrument according to a second implementation mode of the present application;
FIG. 5 is a schematic diagram of a first detachable structure in FIG. 4;
FIG. 6 is a schematic diagram of a reusable ultrasonic surgical instrument according to a third implementation mode of the present application;
FIG. 7 is a schematic diagram of a first detachable structure and a second detachable structure in FIG. 6; and
FIG. 8 is a schematic diagram of a deformation structure of a reusable ultrasonic surgical instrument according to a third implementation mode of the present application.

### DESCRIPTION OF THE EMBODIMENTS

The following clearly and completely describes the technical schemes in the embodiments of the present invention, apparently, the described embodiments are merely some of the embodiments of the present invention rather than all of the embodiments. All other embodiments obtained by a person of ordinary skill in the art based on the embodiments of the present invention without creative efforts shall fall within the protection scope of the present disclosure.

For the convenience of description, "proximal end" in the whole application refers to an end close to an operator after the operator holds an instrument, and "distal end" refers to an end far away from the operator after the operator holds the instrument.

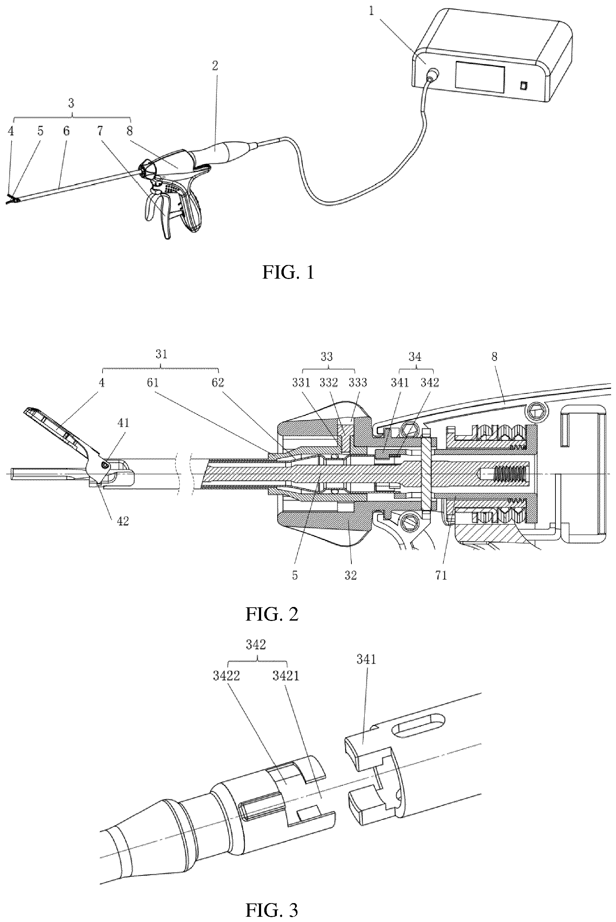

Refer to FIG. 1, an ultrasonic scalpel system is shown, including an ultrasonic generator 1, a transducer 2 and a surgical instrument 3. The ultrasonic generator 1 emits an oscillating electric signal and transmits to the transducer 2, the transducer 2 converts the oscillating electric signal into mechanical vibration and transmits to the surgical instrument 3, and the surgical instrument 3 performs cutting and coagulation on a tissue by utilizing the mechanical vibration of the transducer. Refer to FIG. 1 and 2, a surgical instrument 3 generally includes a scalpel waveguide 5, a pair of clamp forceps 4 forming a clamping structure with a scalpel head (a cutting portion at the head of the scalpel bar), a cannula 6 encircling the outside of the scalpel bar 5, a grab handle 8 and a grasping mechanism 7. The scalpel bar 5 transmits mechanical vibration of the transducer 2 to the scalpel head; the scalpel head is matched with the clamp forceps 4 to clamp a tissue and perform ultrasonic cutting and hemostasis on the clamped tissue; the cannula 6 isolates the scalpel bar 5 from the outside to play a role of protecting the scalpel bar on one hand, and forms a link mechanism with the clamp forceps 4 at a distal end to drive the closing and opening of the clamp forceps 4 on the other hand; the grab handle 8 and the grasping mechanism 7 are held by the hand of a doctor to operate the clamp forceps 4 to open and close, and a switch is provided to control the ultrasonic generator 1 to start to output an oscillating electric signal or stopping outputting an oscillating electric signal.

Refer to FIG. 2 and 3 below, a reusable ultrasonic surgical instrument according to a first implementation mode of the present application is described in details. As shown in FIG. 2, the reusable ultrasonic surgical instrument 3 includes a cannula subassembly 31, a scalpel waveguide 5, a base 32 and a clamping driving component 71, the base 32 is provided at a distal end of the grab handle 8, and the cannula subassembly 31 may be detachably connected with the base 32 and the clamping driving component 71. The cannula subassembly 31 includes a pair of clamping forceps 4 located at a distal end, an external cannula 61 and an internal cannula 62. Two rotating shafts 41 and 42 are provided at the proximal end of the clamping forceps 4, the rotating shaft 41 is rotatably connected with the distal end of the external cannula 61, and the rotating shaft 42 is rotatably connected with the distal end of the internal cannula 62. The internal cannula 61 is provided outside the scalpel bar 5, the external cannula 61 is provided outside the internal cannula 62, and the external cannula 61 and the internal cannula 62 are coaxial with the scalpel bar 5. By the foregoing settings, when the external cannula 61 is fixed, the rotating shaft 41 is also fixed, and the internal cannula 62 is pulled back and forth along the axis of the scalpel bar 5 to drive the rotating shaft 42 to move back and forth, so that the clamping forceps 4 rotate around the rotating shaft 41, to realize opening and closing between the clamp forceps 4 and the scalpel head.

The cannula subassembly 31 may be dismounted from the base 32 and the clamping driving component 71 along a distal end direction of the axis of the scalpel bar 5, and may be also mounted on the base 32 and the clamping driving component 71 along a proximal end direction of the axis of the scalpel bar 5. Such function is mainly implemented by a first detachable structure 33 and a second detachable structure 34, wherein the first detachable structure 33 is a connecting structure between the external cannula 61 and the base 32, and the second detachable structure 34 is a connecting structure between the internal cannula 62 and the clamping driving component 71. Movement of the base 32 along the axis direction of the scalpel bar 5 relative to the grab handle 8 is fixed, and the clamping driving component 71 is connected with the grasping mechanism 7 and is driven by the grasping mechanism 7 to move back and forth along the axis direction of the scalpel bar 5. Therefore, after the external cannula 61 is connected to the base 32 by the first detachable structure 33, and the internal cannula 62 is connected to the clamping driving component 71 by the second detachable structure 34, the clamping driving component 71 can drive the internal cannula 62 to move back and forth along the axis direction of the scalpel bar 5 relative to the base 32, so as to drive the clamp forceps 4 to open or close relative to the head of the scalpel bar 5.

Refer to FIG. 2, in the reusable ultrasonic surgical instrument according to the first implementation mode of the present application, the first detachable structure 33 is formed by an unthreaded hole 333 in the base 32, a threaded hole 331 in a proximal end of the external cannula 61 and a bolt 332. The bolt 332 penetrates through the unthreaded hole 333 to be screwed into the threaded hole 331, in this way, the external cannula 61 and the base 32 are restrained together in the axis direction and the circumferential direction of the scalpel bar 5. A person skilled in the art may easily think that the unthreaded hole 333 may be also provided at the proximal end of the external cannula 61, while the threaded hole 331 is provided on the base 32.

Refer to FIG. 2 and 3, the second detachable structure 34 is formed by a second clamping slot 342 and a hasp structure 341, wherein the second clamping slot 342 is provided at the proximal end of the internal cannula 62, and the hasp structure 341 is provided at the distal end of the clamping driving component 71. Refer to FIG. 3, a second detachable structure 34 in the present implementation mode is shown in details, the second clamping slot 342 is formed by a second sliding slot 3421 and a second stop slot 3422, the second sliding slot 3421 is parallel to the axis direction of the scalpel bar 5 and starts from a nearest end portion of the scalpel bar 5, and the second stop slot 3422 extends by being vertical to the axis direction of the scalpel bar 5 and communicates with the distal end of the second sliding slot 3421; the hasp structure 341 provided at the distal end of the clamping driving component 71 is parallel to the axis direction of the scalpel bar 5, the width of the hasp structure 341 is equal to or slightly smaller than the width of the second sliding slot 3421, the hasp structure 341 has a hooked bulge inwards by being vertical to the axis direction of the scalpel bar 5 and is capable of sliding into the second sliding slot 3421 to enter the second stop slot 3422, and the hasp structure 341 can be buckled on the second stop slot 3422 after rotating by a set mounting angle relative to the second clamping slot 342, so that the movement of the hasp structure 341 along the axis direction of the scalpel bar 5 is restrained by the second stop slot 3422, and the hasp structure 341 can drive the second clamping slot 342 to move along the axis direction of the scalpel bar 5.

When the cannula subassembly 31 is connected with the base 32 and the clamping driving component 71, firstly, the hasp structure 341 on the driving component 71 slides into the second sliding slot 3421 at the proximal end of the internal cannula 62 to enter the second stop slot 3422, then the cannula subassembly 31 is rotated by a set mounting angle so that the hasp structure 341 is buckled on the second stop slot 3422, so that the internal cannula 62 is connected with the clamping driving component 71, and then the bolt 332 penetrates through the unthreaded hole 333 in the base 32 to be screwed in the threaded hole 331 in the external cannula 61, to connect the external cannula 61 with the base 32. When the cannula subassembly 31 is dismounted from the base 32 and the clamping driving component 71, firstly, the bolt 332 of the first detachable structure 33 is screwed out from the threaded hole 331 and withdrawn from the unthreaded hole 333, then the cannula subassembly 31 is rotated by a set mounting angle, so that the hasp structure 341 is rotated to the second sliding slot 3421 from the second stop slot 3422, and finally, the cannula subassembly 31 is moved out towards the distal end direction of the axis of the scalpel bar 5, thereby completing dismounting of the cannula subassembly 31 from the base 32 and the clamping driving component 71.

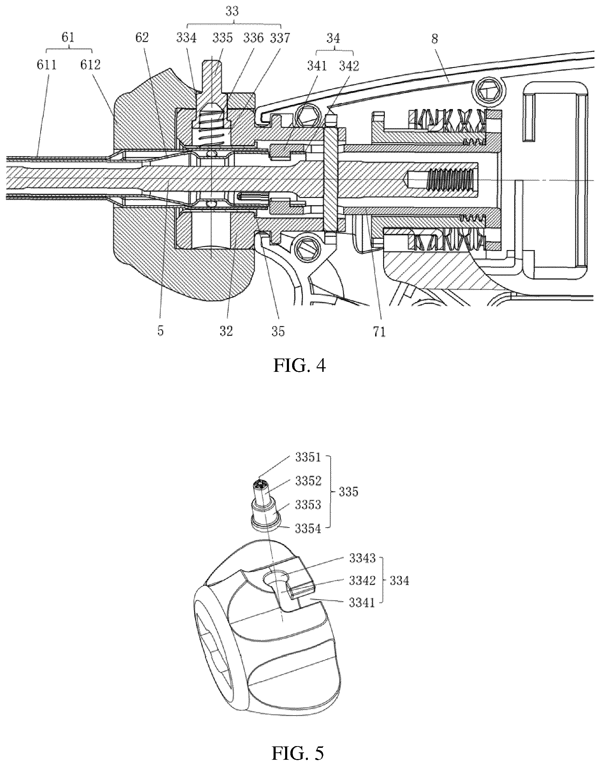

Refer to FIG. 4 and 5 below, a reusable ultrasonic surgical instrument according to a second implementation mode of the present application is described in details. In the present application mode, most features are similar to those in the first implementation mode, and are not repeated herein, moreover, the second detachable structure 34 in FIG. 4 is similar to that in the embodiment in FIG. 2, and a difference between FIG. 4 and FIG. 2 only lies in the construction of the first detachable structure 33. Refer to FIG. 4, the external cannula 61 is formed by a cannula main body 611 and a clamping seat 612 located at the proximal end of the cannula main body 611, wherein the cannula main body 611 is made from a metal material, the clamping seat 612 is made from a metal or nonmetal material, and the two may be bonded together by a mode of welding, adhering or co-casting. The first detachable structure 33 is formed by a first clamping slot 334, a button 335, an elastic element 336 and a clamping seat 337. The first clamping slot 334 is provided on the clamping seat 612, and the button 335, the elastic element 336 and the clamping seat 337 are provided on the base 32. FIG. 5 shows a detailed structure of the first clamping slot 334 and the button 335, wherein the first clamping slot 334 is formed by a first sliding slot presenting an L shape on the whole and a first stop slot 3343 connected with the first sliding slot, the first sliding slot is formed by a first portion 3341 parallel to the axis of the scalpel bar 5 and a second portion 3342 vertical to the axis of the scalpel bar 5, one end of the first portion 3341 starts from a proximal end side of the base 32, the other end is connected with an end portion of the second portion 3342, the other end of the second portion 3342 is connected with the first stop slot 3343, and the maximum width of the first stop slot 3343 is greater than the maximum width of the first sliding slot. The button 335 has a button axis 3351, and the elastic element 336 is provided below the button 335 so that the button 335 can move between a highest position and a lowest position along the direction of the button axis 3351. The button 335 is formed by mutual connection of a mounting section 3354, a stop section 3353 and a dismounting section 3352 which are distributed along the direction of the button axis 3351, the maximum width of a cross section of the mounting section 3354 vertical to the direction of the button axis 3351 is greater than the maximum width of a cross section of the stop section 3353 vertical to the direction of the button axis 3352, and the maximum width of a cross section of the stop section 3353 vertical to the direction of the button axis 3351 is greater than the maximum width of a cross section of the dismounting section 3352 vertical to the direction of the button axis 3351. The mounting section 3354 located at the lowest end of the button 335 is connected with a clamping seat 337 and an elastic element 336 on the base 32. The stop section 3353 located above the mounting section 3354 can be embedded into the first stop slot 3343 on the clamping seat 612, and can be restrained by the first stop slot 3343 in movement vertical to the direction of the button axis 3351. The maximum width of a cross section of the dismounting section 3352 located above the stop section 3353 vertical to the direction of the button axis 3351 is smaller than the minimum width of the first clamping slot 334, and thus the dismounting section 3352 can freely slide in the first clamping slot 334. The clamping seat 337 is a structure on the base 32, and the mounting section 3354 of the button 335 and the elastic element 336 are mounted in the clamping seat 337. The elastic element 336 may be a cylinder spring, a wave spring or other elastomers, one end of the elastic element 336 is pressed on the bottom of the mounting section 3354 of the button 335, and the other end is pressed on a cylindrical component 35 sealing a hole in the bottom of the clamping seat 337. Suffering from the elastic force of the elastic element 336, the button 335 is in a highest position in a natural state, at the moment, if the stop section 3353 of the button 335 is embedded into the first stop slot 3343, fixing of the external cannula 61 along the axis and the circumferential direction of the scalpel bar 5 may be realized. When an external force presses the button 335 to cause the button 335 to move to a lowest position by overcoming the elastic force of the elastic element 336, the dismounting section 3352 of the button 335 is located in the first clamping slot 334, and then the button 335 can move in the first clamping slot 334 and can slide out sequentially from the first stop slot 3343, the second portion 3342 vertical to the axis of the scalpel bar 5 of the first sliding slot and the first portion 3341 parallel to the axis of the scalpel bar 5 of the first sliding slot, so that the external cannula 61 is separated from the base 32.

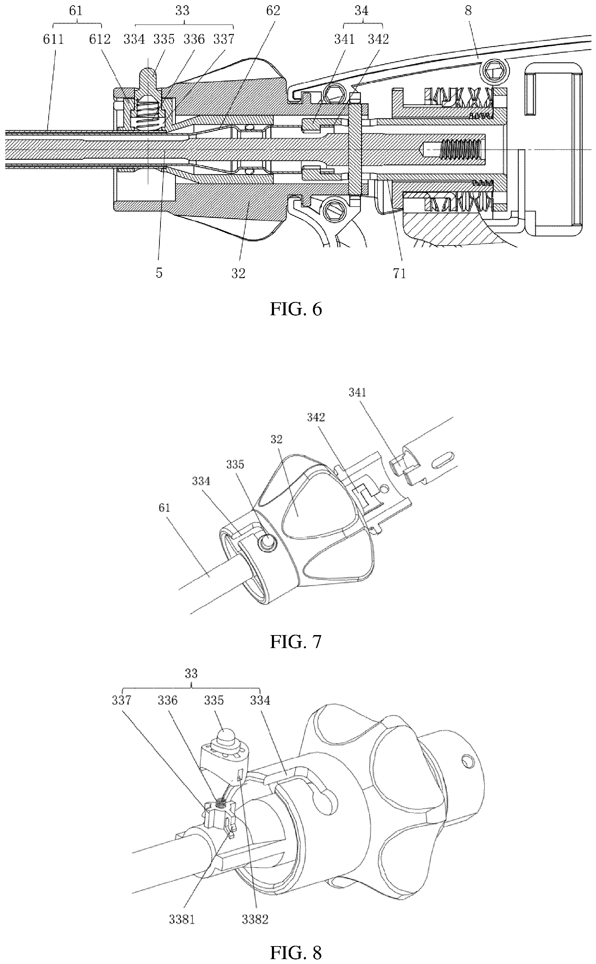

Refer to FIG. 6 and 7 below, a reusable ultrasonic surgical instrument according to a third implementation mode of the present application is described in details. In the present implementation mode, most features are similar to those in the second implementation mode, and are not repeated herein, and a difference between the present implementation mode and the second implementation mode shown in FIG. 4, 5 only lies in that a setting object of the first detachable structure 33 is different. Refer to FIG. 6 and 7, the external cannula 61 is formed by a cannula main body 611 and a clamping seat 612 located at the proximal end of the cannula main body 611, wherein the cannula main body 611 is made from a metal material, the clamping seat 612 is made from a metal or nonmetal material, and the two may be bonded together by a mode of welding, adhering or co-casting. The first detachable structure 33 is formed by a first clamping slot 334, a button 335, an elastic element 336 and a clamping seat 337. The first clamping slot 334 is provided on the base 32, and the button 335, the elastic element 336 and the clamping seat 337 are provided on the clamping seat 612. Other structures and mounting and dismounting modes are similar to those in FIG. 4 and 5, and are not repeated herein.

FIG. 8 is a schematic diagram of a deformation structure of a first detachable structure 33 according to a third embodiment. In such structure, the first detachable structure 33 is formed by a first clamping slot 334, a button 335, an elastic element 336 and a clamping seat 337. The button 335 is connected with the clamping seat 337 by a buckle structure, a buckle 3381 is provided on the clamping seat 337, and a buttonhole 3382 is provided on the button 335. The elastic element 336 is mounted into the clamping seat 337, and then the button 335 is sleeved into the clamping seat 337, so that the buckle 3381 is buckled into the buttonhole 3382, thus completing connection between the button 335 and the clamping seat 337. The height of the buttonhole 3382 is greater than the height of the buckle 3381, so that the buckle 3381 can move in the buttonhole 3382 along the button axis direction, and then the button 335 can move between a highest position and a lowest position.

The reusable ultrasonic surgical instrument according to the present application solves the problems that mainstream ultrasonic surgical instruments on the market are difficult to clean after use, and cannot be repeatedly used, and can remarkably lower the use cost of the instrument. Moreover, in comparison with reusable ultrasonic systems in the prior art, reusable components in the reusable ultrasonic surgical instrument of the present application are increased, so as to further lower the use cost, moreover, convenience in mounting and dismounting, reliability of the overall structure, and simplicity in implementation of the process are all promoted. In brief, the present application has the advantages of being simple in stricture and low in cost in comparison with the prior art.

It should to be noted that implementation schemes in the accompanying drawings are merely representative embodiments of the present application, a person skilled in the art may easily understand that the protection scope of the present application is not merely limited in a scope defined by implementation modes in the accompanying drawings, and combination, transformation and variation for implementation modes in the drawings all fall within the protection scope of the present application.

The foregoing disclosed are merely several preferred embodiments of the present application, of course, the protection scope of the present application should be not limited hereby, therefore, equivalent variations made according to claims of the present application still belong to a coverage scope of the present application.

## Claims

1. A reusable ultrasonic surgical instrument, comprising a cannula subassembly, a scalpel waveguide, a base and a clamping driving component, the cannula subassembly comprising a pair of clamp forceps at a distal end, an external cannula and an internal cannula, and the external cannula and the internal cannula being both provided by being coaxial with the scalpel bar; two rotating shafts being provided at a proximal end of the clamp forceps, one of the two rotating shafts being rotatably connected with the external cannula, the other rotating shaft being rotatably connected with the internal cannula; the cannula subassembly being dismounted from the base and the clamping driving component towards a distal end direction of the axis of the scalpel bar or being mounted on the base or the clamping driving component towards a proximal end direction of the axis of the scalpel bar; the external cannula being connected with the base by one or multiple first detachable structures; and the internal cannula being connected with the clamping driving component by one or multiple second detachable structures.

2. The reusable ultrasonic surgical instrument according to claim 1, wherein the first detachable structure is formed by an unthreaded hole in the base, a threaded hole in the external cannula and a bolt, the bolt being capable of penetrating through the unthreaded hole in the base to be screwed into or screwed out from the threaded hole in the external cannula.

3. The reusable ultrasonic surgical instrument according to claim 1, wherein the first detachable structure is formed in a threaded hole in the base, an unthreaded hole in the external cannula and a bolt, the bolt being screwed into or screwed out from the threaded hole in the base to penetrate into or withdraw from the unthreaded hole in the external cannula.

4. The reusable ultrasonic surgical instrument according to claim 1, wherein the first detachable structure is formed by a first clamping slot, a button, an elastic element and a clamping seat; the first clamping slot being formed by mutual connection of a first sliding slot and a first stop slot; the button having a button axis, and comprising a mounting section, a stop section and a dismounting section which are distributed along the axis direction of the button and are connected with one another; the elastic element being provided in the button axis direction of the button so that the button can move between a highest position and a lowest position along the button axis direction; the mounting section of the button being connected with the clamping seat and the elastic element, so that the button is in the highest position in a natural state, and can move to the lowest position by overcoming the elastic movement of the elastic element under the action of an external force; the stop section of the button being embedded into the first stop slot, and being restrained by the first stop slot in movement vertical to the direction of the button axis; and the dismounting section of the button being capable of freely sliding in the first clamping slot.

5. The reusable ultrasonic surgical instrument according to claim 4, wherein the first clamping slot is provided on the external cannula, and the button, the elastic element and the clamping seat are provided on the base.

6. The reusable ultrasonic surgical instrument according to claim 4, wherein the first clamping slot is provided on the base, and the button, the elastic element and the clamping seat are provided on the external cannula.

7. The reusable ultrasonic surgical instrument according to claim 5, wherein the clamping seat is a hole with a certain height in the base, and the mounting section of the button is mounted in the clamping seat by the elastic element and can move between a highest position and a lowest position along the hole.

8. The reusable ultrasonic surgical instrument according to claim 6, wherein the clamping seat is a hole with a certain height in the external cannula, and the mounting section of the button is mounted in the clamping seat by the elastic element and can move between a highest position and a lowest position along the hole.

9. The reusable ultrasonic surgical instrument according to claim 7 or 8, wherein one end of the elastic element is pressed at the bottom of the mounting section of the button, and the other end is pressed on a cylindrical component sealing the bottom of the clamping seat.

10. The reusable ultrasonic surgical instrument according to claim 5 or 6, wherein the button is connected with the clamping seat by a structure of a buckle and a buttonhole, the height of the buttonhole being greater than the height of the buckle, so that the buckle can move in the buttonhole along the button axis direction, and then the button can move between a highest position and a lowest position.

11. The reusable ultrasonic surgical instrument according to claim 10, wherein the buckle is provided on the clamping seat, and the buttonhole is formed in the button.

12. The reusable ultrasonic surgical instrument according to claim 10, wherein the buckle is provided on the button, and the buttonhole is formed in the clamping seat.

13. The reusable ultrasonic surgical instrument according to any one of claims 4-8, 11, 12, wherein the elastic element is a cylinder spring or a wave spring.

14. The reusable ultrasonic surgical instrument according to any one of claims 4-8, 11, 12, wherein a part of the first sliding slot is parallel to the axis of the scalpel bar, and the other part is vertical to the axis of the scalpel bar; and the maximum width of the first stop slot is greater than the maximum width of the first sliding slot.

15. The reusable ultrasonic surgical instrument according to claim 1, wherein the second detachable structure is formed by a second clamping slot and a hasp structure; the second clamping slot being formed by mutual connection of a second sliding slot and a second stop slot; and the hasp structure being capable of freely sliding in the second sliding slot and sliding into the second stop slot from the second sliding slot, and being restrained by the second stop slot in movement along the axis direction of the scalpel bar.

16. The reusable ultrasonic surgical instrument according to claim 15, wherein the second clamping slot is located on the internal cannula, and the hasp structure is located on the clamping driving component.

17. The reusable ultrasonic surgical instrument according to claim 15, wherein the second clamping slot is located on the clamping driving component, and the hasp structure is located on the internal cannula.

18. The reusable ultrasonic surgical instrument according to claim 16 or claim 17, wherein the second sliding slot is parallel to the axis of the scalpel bar.

19. The reusable ultrasonic surgical instrument according to claim 16 or claim 17, wherein the second stop slot is vertical to the axis of the scalpel bar.
